(19) Europäisches Patentamt
European Patent Office
Office européen des brevets

(11) Veröffentlichungsnummer: **0 032 990**
**A1**

(12) # EUROPÄISCHE PATENTANMELDUNG

(21) Anmeldenummer: **80107593.8**

(22) Anmeldetag: **04.12.80**

(51) Int. Cl.³: **C 07 D 301/16**
C 07 D 303/04, C 07 D 303/06

(30) Priorität: **26.01.80 DE 3002785**

(43) Veröffentlichungstag der Anmeldung:
**05.08.81 Patentblatt 81/31**

(84) Benannte Vertragsstaaten:
**AT BE CH FR GB IT LI NL SE**

(71) Anmelder: **Degussa Aktiengesellschaft**
**Degussa AG Fachbereich Patente Rodenbacher Chaussee 4 Postfach 1345**
**D-6450 Hanau 1 (Stadtteil Wolfgang)(DE)**

(71) Anmelder: **Henkel Kommanditgesellschaft auf Aktien**
**-Patentabteilung- Postfach 1100 Henkelstrasse 67**
**D-4000 Düsseldorf 1(DE)**

(72) Erfinder: **Käbisch, Gerhard, Dr.**
**Palmstrasse 1**
**D-7888 Rheinfelden 5(DE)**

(72) Erfinder: **Trübe, Rudolf**
**Ernst-Reuter-Strasse 22**
**D-7888 Rheinfelden(DE)**

(72) Erfinder: **Wittmann, Hans**
**Katzenbuckelweg 12**
**D-7888 Rheinfelden 8(DE)**

(72) Erfinder: **Raupach, Siegfried**
**Langentalstrasse 24**
**D-7888 Rheinfelden(DE)**

(72) Erfinder: **Malitius, Horst**
**Blümleacker 5**
**D-7888 Rheinfelden(DE)**

(54) **Verfahren zur Epoxydierung olefinisch ungesättigter Verbindungen.**

(57) Olefinisch ungesättigte Verbindungen werden durch Umsetzung mit in situ gebildeter Perameisensäure in die entsprechenden Epoxide umgewandelt. Sie werden zusammen mit Ameisensäure einer Konzentration von mehr als 70 Gewichtsprozent in Mengen zwischen 0,2 und 1,0 Mol pro Mol Doppelbindung vorgelegt. Bei Temperaturen unter 50°C wird Wasserstoffperoxid einer Konzentration von mehr als 50 Gewichtsprozent in Mengen zwischen 1,0 und 1,7 Mol pro Mol Doppelbindung zudosiert. Während der gesamten Dauer der Umsetzung wird in der wässrigen Phase ein pH zwischen 1 und 4 eine hohe Konzentration an einem unter den Reaktionsbedingungen inerten, neutralen, anorganischen Salz aufrecht erhalten.

EP 0 032 990 A1

Degussa Aktiengesellschaft
Weissfrauenstrasse 9, 6000 Frankfurt 1

Henkel Kommanditgesellschaft auf Aktien,
Henkelstrasse 67, 4000 Düsseldorf

Beschreibung

## Verfahren zur Epoxydierung olefinisch ungesättigter Verbindungen

Die Erfindung betrifft ein Verfahren zur Epoxydierung olefinisch ungesättigter Verbindungen durch Umsetzung mit in situ aus Ameisensäure und Wasserstoffperoxid gebildeter Perameisensäure.

Es ist bereits seit langem bekannt, die Ester höherer ungesättigter Fettsäuren mit innenständiger Doppelbindung, beispielsweise Sojabohnenöl, durch Umsetzung mit in situ aus Ameisensäure und Wasserstoffperoxid gebildeter Perameisensäure zu epoxydieren (vgl. z.B. US-PS 2 485 160).

Eine Übertragung der dabei gewonnenen Erkenntnisse auf die Epoxydierung von schwieriger zu epoxydierenden olefinisch ungesättigten Verbindungen scheiterte an den nur schlechten Ausbeuten. Infolgedessen wurde in der Literatur übereinstimmend immer wieder darauf hingewiesen, dass die weit überwiegende Mehrzahl der bekannten olefinisch ungesättigten Verbindungen mit in situ-Perameisensäure praktisch nicht epoxydierbar ist (vgl. z.B. Chemical Week vom 6. April 1963, Übersicht auf Seite 60).

Im Laufe der Zeit wurden zwar zahlreiche Verfahren zur Epoxydierung bestimmter Olefine oder bestimmter Klassen von Olefinen entwickelt. Bei diesen Verfahren werden verschiedenartige Persäuren verwendet, teils vorgefertigt, teils in situ gebildet, und es kommen die unterschiedlichsten Reaktionsbedingungen zur Anwendung. Ein allgemein anwendbares Verfahren zur Epoxydierung von olefinisch ungesättigten Verbindungen mit der einfachsten - und zugleich wirtschaftlich günstigsten - Percarbonsäure, der in situ gebildeten Perameisensäure, existiert jedoch bis heute nicht.

Das erfindungsgemässe Verfahren ist dadurch gekennzeichnet, dass man die olefinisch ungesättigte Verbindung zusammen mit Ameisensäure einer Konzentration von mehr als 70 Gewichtsprozent in Mengen zwischen 0,2 und 1,0 Mol pro Mol zu epoxydierender Doppelbindung vorlegt, bei Temperaturen unter 50°C Wasserstoffperoxid einer Konzentration von mehr als 50 Gewichtsprozent in Mengen zwischen 1,0 und 1,7 Mol pro Mol zu epoxydierender Doppelbindung zudosiert und während der gesamten Dauer der Umsetzung in der

wässrigen Phase einen pH zwischen 1 und 4 und eine hohe Konzentration an einem unter den Reaktionsbedingungen inerten, neutralen, anorganischen Salz aufrecht erhält.

Das erfindungsgemässe Verfahren lässt sich ganz allgemein zur Epoxydierung olefinisch ungesättigter Verbindungen anwenden. Allerdings können bestimmte Olefine auf kostengünstigere Weise nach Verfahren epoxydiert werden, die entweder schon lange bekannt sind (z.B. Epoxydierung von Sojabohnenöl), oder Gegenstand von nicht vorveröffentlichten Parallelanmeldungen sind.

Es gibt jedoch zahlreiche olefinisch ungesättigte Verbindungen, deren Epoxydierung mit in situ gebildeter Perameisensäure nach anderen Verfahren entweder überhaupt nicht oder nur unter grossen Schwierigkeiten und mit unbefriedigenden Ausbeuten an dem gewünschten Epoxid möglich ist. Beispiele für derartige Olefine sind

unsubstituierte oder substituierte cycloaliphatische Verbindungen mit 1 bis 3 Ringen, die eine oder mehrere Doppelbindungen enthalten, wobei die Doppelbindungen in einem Ring und/oder in einem Substituenten liegen können, wie Cyclohepten, Cyclooctadien, Dicyclopentadien, Bicycloheptadien, Tricyclodecen-8, Tricycloundecen oder Vinylcyclohexen;

als Isoprenabkömmlinge aufzufassende Terpene, wie Myrcen oder Trimethylcyclododecatrien; und

geradkettige oder verzweigte, ein- oder mehrfach ungesättigte aliphatische Olefine mit 8 bis 10 Kohlenstoffatomen und endständigen oder innenständigen Doppelbindungen. Wirtschaftlich wichtige Vertreter aus dieser Gruppe sind beispielsweise Octen-1 und Decen-1.

Zur praktischen Durchführung des erfindungsgemässen Verfahrens wird die zu epoxydierende olefinisch ungesättigte Verbindung in einem kühlbaren Rührgefäss zusammen mit Ameisensäure einer Konzentration von mehr als 70 Gewichtsprozent, vorzugsweise mehr als 84 Gewichtsprozent, in Mengen zwischen 0,2 und 1,0 Mol, vorzugsweise zwischen 0,2 und 0,6 Mol, pro Mol zu epoxydierender Doppelbindung vorgelegt.

Ebenfalls vorgelegt wird ein unter den Reaktionsbedingungen inertes, neutrales, anorganisches Salz. Dessen Menge ist so zu bemessen, dass in der wässrigen Phase auch am Ende der Umsetzung, also nach Zugabe des gesamten Wasserstoffperoxids, noch eine möglichst hohe Konzentration, vorzugsweise Sättigung, gegebenenfalls mit ungelöstem Bodenkörper, herrscht. Geeignete Salze sind alle gegenüber den Einsatzstoffen und den Reaktionsprodukten unter den Reaktionsbedingungen inerten Salze starker anorganischer Säuren mit starken anorganischen Basen, die keine Zersetzung von Wasserstoffperoxid oder Perameisensäure bewirken, wie kristallwasserfreie Alkalisalze der Schwefel- oder Salzsäure. Bevorzugt werden Natriumsulfat, Natriumchlorid oder Kaliumchlorid verwendet. Diese Salze können beispielsweise in Mengen von etwa 20 bis etwa 40 Gewichtsprozent, bezogen auf das Gewicht der wässrigen Phase am Ende der Umsetzung, vorgelegt werden. Ebensogut ist es aber auch möglich, zunächst nur einen Teil des Salzes vorzulegen und den Rest im Verlaufe der Umsetzung zuzugeben.

Zur Einstellung des pH-Wertes in der wässrigen Phase auf den Bereich zwischen 1 und 4, vorzugsweise zwischen 2 und 3 ist es ferner vorteilhaft, ein Alkalimetallsalz einer niederen Carbonsäure zu verwenden. Wird ein solches Salz verwendet, wird es zweckmässigerweise ebenfalls vorgelegt. Die einzusetzende Menge richtet sich nach der eingesetzten Menge an Ameisensäure und dem gewünschten pH. Bevorzugt werden die Natrium- oder Kaliumsalze der aliphatischen Carbonsäuren mit 1 bis 3 Kohlenstoffatomen verwendet. Besonders bevorzugt werden das Natriumformiat und das Natriumacetat, jeweils in wasserfreier Form.
Zu der vorgelegten Mischung wird dann unter Rühren und bei Temperaturen unter $50^{o}C$, vorzugsweise von 20 bis $45^{o}C$, Wasserstoffperoxid einer Konzentration von mehr als 50 Gewichtsprozent, vorzugsweise von mehr als 65 Gewichtsprozent, im Verlauf von mehreren Stunden zudosiert. Die Zeitdauer der Zudosierung des Wasserstoffperoxids hängt insbesondere von der Reaktionsgeschwindigkeit bei der Epoxydierung und der Grösse der zur Verfügung stehenden Wärmeaustauschfläche ab. Das Wasserstoffperoxid wird in Mengen zwischen 1,0 und 1,7 Mol, vorzugsweise zwischen 1,1 und 1,5 Mol, pro Mol zu epoxydierender Doppelbindung eingesetzt.

Das erfindungsgemässe Verfahren ist auch in grosstechnischen Produktionsanlagen gefahrlos durchführbar, wenn einige sicherheitstechnische Massnahmen ergriffen werden. So können z.B. automatische Sicherheitsventile vorgesehen werden, die sich öffnen und den Ansatz mit Wasser verdünnen, wenn die Mischorgane ausfallen, die Menge des durch Zersetzung von Wasserstoffperoxid oder Perameisensäure gebildeten Abgases einen Grenzwert überschreitet

oder die Temperatur merklich über 50°C anzusteigen droht.

Die Umsetzung zwischen der olefinisch ungesättigten Verbindung und der in situ gebildeten Perameisensäure wird vorteilhafterweise drucklos durchgeführt. Sie erfordert im allgemeinen eine Reaktionszeit zwischen 5 und 15 Stunden, insbesondere zwischen 6 und 10 Stunden.

Nach beendeter Epoxydierungsreaktion wird im Normalfall die wässrige Phase von der organischen Epoxidphase getrennt. Zur weiteren Reinigung empfiehlt es sich, die organische Phase von darin noch gelösten Wasserstoffperoxid- und Ameisensäureanteilen zu befreien. Bevorzugt wird hierzu ein zweimal hintereinander vorgenommenes Auswaschen mit Wasser. Das nach dem letzten Auswaschen in der organischen Phase noch gelöste Wasser kann leicht in an sich bekannter Weise, z.B. durch leichtes Erwärmen unter vermindertem Druck, destillativ entfernt werden.

Die nach dem erfindungsgemässen Verfahren hergestellten Epoxide sind häufig so rein, dass sie für die meisten Verwendungszwecke direkt eingesetzt werden können. Sollte dies in speziellen Fällen erwünscht sein, können sie aber natürlich auch in an sich bekannter Weise, z.B. durch Totaldestillation unter vermindertem Druck, weitergereinigt werden.

Das erfindungsgemässe Verfahren soll durch die nachfolgenden Beispiele näher erläutert werden. Die Prozentangaben bedeuten, sofern nicht anders angegeben, Gewichtsprozente.

Beispiel 1:

In einem auf 35 bis 40°C temperierten Rührkolben wurden vermischt

| | | | |
|---|---|---|---|
| Trimethylcyclododecatrien (technisch) | 1020 g | = | 5,00 Mol |
| Ameisensäure (98 %ig) | 115 g | = | 2,50 Mol |
| Natriumsulfat (wasserfrei) | 60 g | = | 0,42 Mol |
| Natriumformiat | 38 g | = | 0,56 Mol |

Unter kräftigem Rühren wurden im Verlauf von 3 1/2 Stunden 240 g $H_2O_2$ (85 %ig) = 6,0 Mol gleichmässig eindosiert. In der wässrigen Phase wurde während der Umsetzung ein pH-Wert gemessen, der bei 2,5 lag.

Nach beendeter $H_2O_2$-Eindosierung wurde der Ansatz 2 Stunden bei 40°C gerührt, zweimal mit je 500 ml Wasser gewaschen und bei 60°C (12 Torr) von gelöstem Wasser befreit.
Die Untersuchung der organischen Phase ergab folgende Zusammensetzung:

| | |
|---|---|
| Kohlenwasserstoffverunreinigungen, die schon zu Beginn im technischen Ausgangsprodukt enthalten waren | 9,0 % |
| Trimethylcyclododecatrien | 10,3 % |
| " -Monoepoxid | 72,1 % |
| " -Diepoxid | 8,6 % |

Die angegebene Monoepoxid-Ausbeute erhöht sich auf 79,2 %, wenn man die im technischen Ausgangsprodukt enthaltenen Verunreinigungen rechnerisch berücksichtigt. Bezieht man die zuletzt angegebene Ausbeute darüber hinaus auf umgesetztes Trimethylcyclododecatrien, dann ergibt sich ein Wert von 89,3 % Monoepoxid.

## Beispiel 2:

In einem auf 35 bis 38 °C temperierten Rührkolben wurden vermischt

| | | | |
|---|---|---|---|
| Cyclohepten (technisch) | 481 g | = | 5,00 Mol |
| Ameisensäure (98 %ig) | 115 g | = | 2,50 Mol |
| Natriumsulfat (wasserfrei) | 60 g | = | 0,42 Mol |
| Natriumformiat | 38 g | = | 0,56 Mol |

Unter kräftigem Rühren wurden im Verlauf von 4 Stunden 240 g $H_2O_2$ (85 %ig) = 6,00 Mol gleichmässig eindosiert. In der wässrigen Phase wurde während der Umsetzung ein pH-Wert gemessen, der bei 2,5 lag. Nach beendeter $H_2O_2$-Eindosierung wurde der Ansatz noch 3 Stunden lang bei 37 °C weitergerührt, zweimal mit je 100 ml Wasser gewaschen, einmal mit 100 ml $FeSO_4$-Lösung (5 %ig) das vorhandene Cycloheptenperoxid zerstört, die organische Phase mit $Na_2SO_4$ (wasserfrei) getrocknet und filtriert. Die gaschromatographische Untersuchung ergab folgende Zusammensetzung:

| | |
|---|---|
| Kohlenwasserstoffverunreinigungen, die schon zu Beginn im technischen Ausgangsprodukt enthalten waren | 28,9 % |
| Cyclohepten | 8,5 % |
| " -oxid | 57,7 % |
| hochsiedende Nebenprodukte | 4,9 % |

Die angegebene Epoxid-Ausbeute erhöht sich auf 87,2 %, wenn man die im technischen Ausgangsprodukt enthaltenen Verunreinigungen rechnerisch berücksichtigt. Bezieht man die zuletzt angegebene Ausbeute darüberhinaus auf umgesetztes Cyclohepten, dann ergibt sich ein Wert von 92,2 % Epoxid. Das Epoxydierungsprodukt obiger Zusammensetzung lässt sich destillativ sehr einfach trennen. Das erhaltene Cycloheptenoxid siedet bei 82,5° C (65 Torr), ist im Reinheitsgrad > 99 %ig und hat eine Refraktion $n_D^{20} = 1,4652$.

Beispiel 3:

Bei einer Temperatur von 32 bis 35°C wurden in einem temperierten Rührkolben

134,2 g (1,0 Mol) Tricyclodecen-8 der Formel

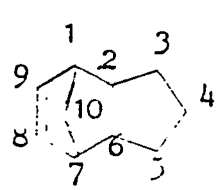

| | | |
|---|---|---|
| 23,0 g | (0,5 Mol) | Ameisensäure (98 Gewichtsprozent) |
| 12,7 g | | Natriumsulfat (wasserfrei) und |
| 7,0 g | | Natriumformiat |

vermischt.

Unter kräftigem Rühren wurden im Verlauf von 3 Stunden 40 g $H_2O_2$ (85 Gewichtsprozent) = 1,0 Mol gleichmässig eindosiert. Nach beendeter Wasserstoffperoxid-Zudosierung wurde der Ansatz noch 3 Stunden lang bei ca. 33°C gerührt und danach mit 100 ml Wasser versetzt. Nach Durchmischung und Phasentrennung wurden 144 ml wässrige Phase abgezogen, die noch einen Restgehalt von 4,2 g Wasserstoffperoxid enthielt.

Die gaschromatographische Untersuchung der verbleibenden organischen Phase ergab nachstehende Zusammensetzung:

ca. 62 % 8,9-Epoxytricyclodecan
ca. 34 % nicht umgesetztes Olefin und
ca. 4 % nicht identifizierte Nebenprodukte.

<u>Beispiel 4:</u>

132 g (1,0 Mol) technisches Dicyclopentadien (Gehalt ca. 94 %) der Formel

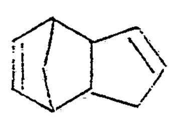

| | | |
|---|---|---|
| 19 g | (0,4 Mol) | Ameisensäure |
| 12 g | | Natriumsulfat (wasserfrei) und |
| 6 g | | Natriumformiat |

wurden bei einer Temperatur von 45 bis 50°C in einem temperierten Rührkolben vermischt. Unter kräftigem Rühren wurden im Verlaufe von 4 Stunden 52 g $H_2O_2$ (85 Gewichtsprozent) = 1,3 Mol gleichmässig eindosiert. Zur Nachreaktion wurde der Ansatz 2 weitere Stunden bei 45 bis 50°C gerührt. Nach dem Abtrennen der wässrigen Phase und zweimaligem Auswaschen der organischen Phase mit je 80 ml Wasser bei einer Temperatur von 80°C hinterblieben 140 g, die nach gaschromatographischer Analyse etwa wie folgt zusammengesetzt waren:

ca. 68 % Monoepoxid-Gemisch aus etwa gleichen Anteilen der beiden Epoxide

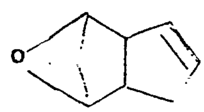

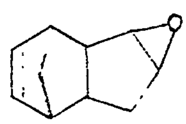

ca. 27 % nicht umgesetztes Olefin mit den Verunreinigungen des Ausgangsproduktes

ca. 5 % nicht identifizierte Nebenprodukte (möglicherweise Diepoxid).

Durch Destillation bzw. Sublimation unter vermindertem Druck kann die organische Phase leicht aufgearbeitet werden. Die dabei zwischen 100 und 110°C bei 10 Torr übergehende Fraktion enthält das Gemisch der beiden Monoepoxide in reiner Form.

Beispiel 5:

Analog wie im Beispiel 4 wurde technisches Myrcen (Gehalt ca. 80 %) der Formel

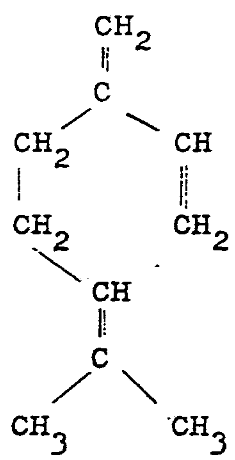

zum Monoepoxid umgesetzt. Die Reaktion wurde jedoch nach ca. 50 %igem Umsatz abgebrochen. Bei der Destillation unter vermindertem Druck von 12 Torr ging das nicht umgesetzte Myrcen bei etwa 60°C und das Myrcenoxid bei etwa 75°C über.

1. Dezember 1980
PAT/Dr.Sib-El

0032990

80 109 A0

Degussa Aktiengesellschaft
Weissfrauenstrasse 9, 6000 Frankfurt 1

Henkel Kommanditgesellschaft auf Aktien
Henkelstrasse 67, 4000 Düsseldorf

Verfahren zur Epoxydierung olefinisch ungesättigter Verbindungen

Patentansprüche:

1. Verfahren zur Epoxydierung olefinisch ungesättigter Verbindungen durch Umsetzung mit in situ aus Ameisensäure und Wasserstoffperoxid gebildeter Perameisensäure, dadurch gekennzeichnet, dass man die olefinisch ungesättigte Verbindung zusammen mit Ameisensäure einer Konzentration von mehr als 70 Gewichtsprozent in Mengen zwischen 0,2 und 1,0 Mol pro Mol zu epoxydierender Doppelbindung vorlegt, bei Temperaturen unter $50^{o}C$ Wasserstoffperoxid einer Konzentration von mehr als 50 Gewichtsprozent in Mengen zwischen 1,0 und 1,7 Mol pro Mol zu epoxydierender Doppelbindung zudosiert und während der gesamten Dauer der Umsetzung in der wässrigen Phase einen pH zwischen 1 und 4 und eine hohe Konzentration an einem unter den Reaktionsbedingungen inerten,

neutralen, anorganischen Salz aufrecht erhält.

2. Verfahren nach Anspruch 1, dadurch gekennzeichnet, dass man Ameisensäure einer Konzentration von mehr als 84 Gewichtsprozent einsetzt.

3. Verfahren nach Anspruch 1 oder 2, dadurch gekennzeichnet, dass man die Ameisensäure in Mengen zwischen 0,2 und 0,6 Mol pro Mol zu epoxydierender Doppelbindung einsetzt.

4. Verfahren nach einem der Ansprüche 1 bis 3, dadurch gekennzeichnet, dass man die Umsetzung bei Temperaturen zwischen 20 und 45°C vornimmt.

5. Verfahren nach einem der Ansprüche 1 bis 4, dadurch gekennzeichnet, dass man Wasserstoffperoxid einer Konzentration von mehr als 65 Gewichtsprozent einsetzt.

6. Verfahren nach einem der Ansprüche 1 bis 5, dadurch gekennzeichnet, dass man das Wasserstoffperoxid in Mengen zwischen 1,1 und 1,5 Mol pro Mol zu epoxydierender Doppelbindung einsetzt.

7. Verfahren nach einem der Ansprüche 1 bis 6, dadurch gekennzeichnet, dass man in der wässrigen Phase einen pH zwischen 2 und 3 aufrecht erhält.

8. Verfahren nach einem der Ansprüche 1 bis 7, dadurch gekennzeichnet, dass man den pH der wässrigen Phase durch den Zusatz eines Alkalimetallsalzes einer niederen Carbonsäure einstellt.

9. Verfahren nach einem der Ansprüche 1 bis 8, dadurch gekennzeichnet, dass man die wässrige Phase an dem unter den Reaktionsbedingungen inerten, neutralen, anorganischen Salz gesättigt hält.

0032990

Europäisches Patentamt

# EUROPÄISCHER RECHERCHENBERICHT

Nummer der Anmeldung

EP 80 10 7593.8

| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | |
| | DE - C - 962 073 (BADISCHE ANILIN- & SODA-FABRIK)<br>* Beispiele *<br>-- | 1,2,4 | C 07 D 301/16<br>C 07 D 303/04<br>C 07 D 303/06 |
| | Chemical Abstracts Band 41, Nr. 21<br>10. November 1947<br>Columbus, Ohio, USA<br>D. SWERN "Electronic interpretation of the reaction of olefins with organic per acids"<br>Spalte 6870f<br>& J. Am. Chem. Soc. Band 69, 1947<br>Seiten 1692 bis 1698<br>-- | 1 | RECHERCHIERTE SACHGEBIETE (Int Cl.³)<br>C 07 D 301/16<br>C 07 D 303/04<br>C 07 D 303/06 |
| | Chemical Abstracts Band 80, Nr. 21,<br>27 Mai 1974<br>Columbus, Ohio, USA<br>C. PANDELE et al. "Polydiene epoxides"<br>Seite 60, Spalte 2, Abstract Nr. 122123q<br>& RO - A - 54 845<br>-- | 1 | |
| | DE - B - 1 252 687 (A. N. SAGREDOS)<br>* Beispiel 1 *<br>-- | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | DE - A - 1 568 016 (ASHLAND OIL AND REFINING CO.)<br>* Anspruch 1 *<br>-- | 1 | |
| A | DE - A - 1 618 625 (LAPORTE CHEMICALS LTD.) -- | | |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 30-04-1981 | FROELICH |

0032990

Europäisches Patentamt

# EUROPÄISCHER RECHERCHENBERICHT

Nummer der Anmeldung

EP 80 10 7593.8
- Seite 2 -

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| D,A | US - A - 2 485 160 (W.D. NIEDERHAUSER et al.)<br>---- | | |
| | | | RECHERCHIERTE SACHGEBIETE (Int. Cl.³) |

EPA Form 1503.2 06.78